# EUROPEAN PATENT APPLICATION

(11) **EP 4 699 613 A1**
(43) Date of publication of application: **25.02.2026**
(21) Application number: 24793012.6
(22) Date of filing: 17.04.2024
(51) Int. Cl.: A61K 38/08, A61P 17/00, A61P 17/02, C07K 7/06, A61K 8/64, A61Q 19/08, A61Q 19/00, A23L 33/18

(54) **NOVEL USE OF CPNE7 PROTEIN-DERIVED PEPTIDE**

(30) Priority: 17.04.2023 KR 20230050377
(71) Applicant: Hysensbio Co., Ltd, Gwacheon-si Gyeonggi-do 13814 (KR)
(72) Inventor: PARK, Joo Hwang, Gwangmyeong-si Gyeonggi-do 14354 (KR); PARK, Yeoung Hyun, Seoul 04378 (KR); HWANG, Geum Bit, Wonju-si Gangwon-do 26417 (KR); PARK, Jeong Min, Seoul 04570 (KR); KIM, Nam Young, Seoul 07029 (KR)
(74) Representative: Mewburn Ellis LLP
(86) International application number: PCT/KR2024/005173
(87) International publication number: WO 2024/219827

(57) **Abstract**

The composition for treating skin aging or skin wounds according to the present invention comprises a peptide consisting of the amino acid sequence of General Formula 1 below: K-Y-R1-R2-R3-R4-R5-R6-R7-R8 (General Formula 1). In General Formula 1 above, R1 is arginine (R), lysine (K), or glutamine (Q); R2 is arginine (R) or glutamine (Q); R3, R4, and R5 are each arginine (R) or lysine (K); R6 is asparagine (N) or serine (S); and R7 and R8 are lysine (K) or tyrosine (Y).

## Description

### [Technical Field]

The present invention relates to a novel use of peptides, and more specifically, to a novel use of peptides derived from CPNE7 protein that promote skin regeneration, elasticity enhancement, and moisturizing ability.

### [Background Art]

The skin is the largest tissue that exists in the outermost layer of our body and is simultaneously distributed throughout the entire body. Therefore, it is exposed to various stimuli including pressure, impact, friction, and others from the external environment. The skin corresponds to an important tissue that protects internal organs from such external environments, regulates body temperature, and enables sensation.

The structure of skin is divided into epidermis, dermis, and subcutaneous fat layer. The subcutaneous fat layer is composed of fat cells below the dermis and is a fat layer where excess nutrients from consumed food are accumulated in the form of fat. It is located between the skin and muscles and has insulation effects, performing the function of maintaining body temperature through this mechanism, thus being distinguished from the dermal layer. The externally observed epidermis is the layer that directly contacts the external environment at the outermost part of the skin and is mainly composed of the stratum corneum. The stratum corneum functions as a barrier that protects our body, preventing even a single drop of water, bacteria, or viruses from penetrating from the outside. The dermis is the layer located directly beneath the epidermis and mainly consists of fiber components such as collagen fibers and elastic fibers, with blood vessels, hair follicles, nerves, and sweat glands existing in between. The dermis provides strength (tension) to the skin and is an important region for forming the skin's shape. When collagen fibers or elastic fibers within the dermal layer become weak or thin, wrinkles form and skin elasticity decreases.

The skin is both a protective membrane with various physiological functions and an important tissue for obtaining aesthetic appeal from others. Therefore, aged skin is accompanied by functional disorders in addition to visible changes. Moreover, severe skin aging has psychological effects on vitality of life and can cause depression in severe cases. Prevention or treatment of skin aging is increasingly important in modern society. Particularly, the increase in average lifespan and decrease in birth rate have brought about an increase in the elderly population. Korea is predicted to reach the super-aged society standard designated by the UN by 2025, so efforts to prevent skin aging cannot be overlooked from a social cost perspective.

The characteristics of skin aging include, first of all, decreased skin thickness. This is particularly prominent in the epidermis, and it has been reported that the reduction in epidermal thickness is accelerated gradually after adulthood. The thickness of the epidermis is known to decrease by approximately 6.4% every 10 years on average. The reduction in epidermal thickness can be a direct pathological cause of skin dryness, as moisture on the skin surface and moisture content in the stratum corneum within the epidermis decrease, and overall lipid content is also known to decrease by approximately 65%. Additionally, as keratinocyte proliferation decreases with age, recovery speed after keratin removal becomes significantly slower in the elderly compared to young people.

Meanwhile, the most prominent structural change inside the skin due to aging is the flattening of the dermo-epidermal junction due to the loss of dermal papillae. Flattening of the dermo-epidermal junction reduces resistance to external friction, making it vulnerable to wound formation. Internally, it reduces the supply of nutrients and oxygen between the dermis and epidermis, disrupts various intercellular signaling systems, and causes separation between the dermis and epidermis. This process is known as one of the major mechanisms of wrinkle formation.

The causes of skin aging can be divided into intrinsic aging and extrinsic aging. Extrinsic aging mainly refers to photoaging caused by ultraviolet rays. However, according to recent studies, it is estimated that various extrinsic factors that promote skin aging, including ultraviolet rays as well as tobacco smoke and pollution, are linked with intrinsic factors to promote skin aging. Intrinsic factors that promote skin aging include genome instability, epigenetic alteration, hormonal changes, or oxidative stress. It has not been clearly revealed at what point genetic level or hormonal changes activate and link these mechanisms. On the other hand, reactive oxygen species (ROS) have been identified as a major cause of overall aging phenomena. It is known through many studies that reactive oxygen species can cause changes in extracellular matrix (ECM) in the dermis in connection with internal and external factors of the skin. Reactive oxygen species naturally generated after external factors such as ultraviolet rays or cellular metabolism bind to cysteine located at the enzyme site within Receptor Protein Tyrosine Phosphatase (RPTP), inhibiting RPTP's ability to inhibit Receptor Tyrosine Kinase (RTK). RTK phosphorylation affects various intracellular signaling systems and induces the activity of Mitogen-Activated Protein Kinase (MAPK) and transcription factors Nuclear Factor-κB (NF-κB) and Activator Protein-1 (AP-1). Activation of NF-κB and AP-1 inhibits collagen production and increases MMP gene transcription, contributing to reducing the amount of collagen in skin tissue and causing abnormal structural characteristics.

Treatment and prevention of skin aging have been a subject of consistent great interest parallel to human history, and research on mechanisms and treatments has continued. However, basic research on skin aging treatment and prevention has not shown outstanding results compared to research on treatments for other diseases. This is also related to not finding fundamental answers to the topic of "cellular aging," which is the higher-level concept of skin aging. While countless studies on fibroblasts in vitro exist, there are also countless studies that have failed to connect research results to anti-aging measures for human skin tissue at the organism level. This suggests that the effects of systemic aging on the skin may be more complex than expected, meaning that research related to skin aging should be viewed from an expanded perspective including not only the skin but also subcutaneous fat, vascular system, nervous system, or immune system.

### [Disclosure]

### [Technical Problem]

The present invention aims to provide peptides and their uses that prevent skin aging and promote skin elasticity by synthesizing Type I Collagen and Type III Collagen in fibroblasts within the dermal layer of the skin.

Another purpose of the present invention is to provide peptides and their uses that prevent skin aging and promote skin elasticity by synthesizing Type IV Collagen in the basement membrane of the dermo-epidermal junction of the skin.

Another purpose of the present invention is to provide peptides and their uses that promote regeneration of the epidermal layer and prevent aging by enhancing cell division and differentiation through increasing Ki-67 expression in keratinocytes within the epidermal layer of the skin.

Another purpose of the present invention is to provide peptides and their uses that promote skin moisturization and prevent aging by enhancing intercellular junction function of keratinocytes through synthesizing Filaggrin and Involucrin in keratinocytes within the epidermal layer of the skin.

Another purpose of the present invention is to provide peptides and their uses that prevent skin aging by inhibiting collagen degradation through reducing MMP-1 expression in keratinocytes and dermal cells within the epidermal and dermal layers of the skin.

The purposes of the present invention are not limited to those mentioned above, and other purposes not mentioned will be clearly understood by those with ordinary skill in the technical field to which the present invention belongs from the description below.

### [Technical Solution]

To solve the above technical problems, the present inventors conducted various studies and developed a composition for inhibiting skin aging or treating wounds containing peptides derived from CPNE7 protein.

As one embodiment to achieve the above-mentioned purposes, the present invention provides a composition for inhibiting skin aging or treating wounds comprising peptides derived from CPNE7 protein having the amino acid sequence of General Formula 1 below.

K-Y-R1-R2-R3-R4-R5-R6-R7-R8 (General Formula 1)

In General Formula 1 above,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are lysine (K) or tyrosine (Y).

The peptides provided by the present invention can provide excellent collagen synthesis ability while not exhibiting cytotoxicity.

The peptides provided by the present invention include variant peptides having sequences that differ by one or more amino acid residues from the amino acid sequences constituting them, as long as they can exhibit skin aging inhibition or wound treatment effects including peptides derived from CPNE7 protein.

Generally, amino acid exchanges in proteins and polypeptides that do not entirely alter the activity of the molecule are known in the art. The most commonly occurring exchanges are between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly. Additionally, peptides with increased structural stability against heat, pH, etc., or increased skin aging inhibition or wound treatment effects by variations or modifications in amino acid sequences may be included.

For example, glutamine, which is a neutral polar amino acid acid at position 3 of the peptide of SEQ ID NO: 1 provided by the present invention, can be substituted with basic amino acids lysine or arginine while still exhibiting the effects of the peptides provided by the present invention. Arginine, which is a basic amino acid at position 4 or 5 of the peptide of SEQ ID NO: 1, can be substituted with acidic amino acid glutamine or basic amino acid lysine while still exhibiting the effects of the peptides provided by the present invention. Lysine, which is a basic amino acid at position 6, 7, or 9 of the peptide of SEQ ID NO: 1, can be substituted with basic amino acid arginine or aromatic amino acid tyrosine while still exhibiting the effects of the peptides provided by the present invention. Asparagine, which is a neutral polar amino acid at position 8 of the peptide of SEQ ID NO: 1, can be substituted with neutral amino acid serine while still exhibiting the effects of the peptides provided by the present invention. Tyrosine, which is an aromatic amino acid at position 10 of the peptide of SEQ ID NO: 1, can be substituted with basic amino acid lysine while still exhibiting the effects of the peptides provided by the present invention.

Thus, even if the acidic amino acids, basic amino acids, or aromatic amino acids constituting the peptides of the present invention are substituted with different acidic amino acids, basic amino acids, neutral amino acids, or aromatic amino acids respectively, they can still exhibit the effects of the peptides provided by the present invention. Therefore, it is obvious that variant peptides having sequences that differ by one or more amino acid residues from the amino acid sequences constituting the peptides of the present invention are also included in the scope of peptides provided by the present invention.

Additionally, even if the peptides of the present invention have a form with arbitrary amino acids added to their N-terminus or C-terminus, they can still exhibit the effects of the peptides provided by the present invention and are therefore included in the scope of peptides provided by the present invention. As one example, they can be in a form with 1 to 300 amino acids added to the N-terminus or C-terminus of the peptide. As another example, they can be in a form with 1 to 100 amino acids added to the N-terminus or C-terminus of the peptide. As yet another example, they can be in a form with 1 to 24 amino acids added to the N-terminus or C-terminus of the peptide.

As another aspect, the present invention provides polynucleotides encoding the above peptides.

The polynucleotides may have one or more bases substituted, deleted, inserted, or combinations thereof. When preparing by chemically synthesizing nucleotide sequences, well-known synthesis methods in the art can be used, for example, methods described in the literature (Engels and Uhlmann, Angew Chem IntEd Engl., 37:73-127, 1988), and synthesis can be performed using triester, phosphite, phosphoramidite and H-phosphate methods, PCR and other auto-primer methods, oligonucleotide synthesis on solid supports, etc. For example, polynucleotides encoding the peptides of the present invention may include the nucleotide sequence of SEQ ID NO: 4.

As yet another aspect, the present invention provides expression vectors containing the above polynucleotides, transformants containing the expression vectors, and methods for producing the peptides using the transformants.

The term "expression vector" in the present invention means a recombinant vector capable of expressing the desired peptide in the desired host cell, and refers to a genetic construct containing essential regulatory elements operably linked so that the gene insert is expressed. The expression vector includes expression control elements such as start codons, stop codons, promoters, operators, etc., where the start codon and stop codon are generally considered part of the nucleotide sequence encoding the polypeptide and must function in the individual when the genetic construct is administered and must be in frame with the coding sequence. The promoter of the vector may be constitutive or inducible.

The term "operably linked" in the present invention means a state where nucleic acid expression control sequences and nucleic acid sequences encoding desired proteins or RNA are functionally linked to perform general functions. For example, a promoter and nucleic acid sequences encoding proteins or RNA can be operably linked to affect expression of the coding sequence. Operative linkage with expression vectors can be prepared using genetic recombination techniques well known in the art, and site-specific DNA cleavage and ligation can use enzymes generally known in the art.

Additionally, the expression vector may include signal sequences for secretion of the peptide to facilitate separation of the peptide from cell culture medium. Specific initiation signals may also be necessary for efficient translation of the inserted nucleic acid sequence. These signals include ATG start codons and adjacent sequences. In some cases, exogenous translation control signals that may include ATG start codons must be provided. These exogenous translation control signals and start codons may be from various natural and synthetic sources. Expression efficiency can be increased by introduction of appropriate transcriptional or translational enhancers.

Furthermore, the expression vector may additionally include protein tags that can be removed using endopeptidases, if desired, to facilitate detection of the peptide.

The term "tag" in the present invention means a molecule showing quantifiable activity or characteristics and may be fluorescent molecules including chemical fluorescent materials such as fluorescein, polypeptide fluorescent materials such as fluorescent proteins (GFP) or related proteins; or may be epitope tags such as Myc tags, Flag tags, histidine tags, leucine tags, IgG tags, streptavidin tags, etc. Particularly, when using epitope tags, preferably peptide tags composed of 6 or more amino acid residues, more preferably composed of 8 to 50 amino acid residues can be used.

The term "photodermatitis" in the present invention may mean a condition where ultraviolet rays or specific substances combined with sunlight cause inflammatory reactions in the skin. One of the major causes of skin damage due to ultraviolet exposure is oxidative stress. Oxidative stress can affect dermal fibroblasts, which can promote collagen degradation and inflammatory reactions. This process can change skin characteristics and worsen symptoms of photodermatitis.

The term "atopic dermatitis" in the present invention is chronic dermatitis characterized by dry and itchy skin with recurring inflammation. It is known to be related to genetic factors, environmental factors, immune system abnormalities, etc. Stress in dermal fibroblasts may be associated with the onset and progression of atopic dermatitis. Oxidative stress generates free radicals that damage cells, causing inflammatory reactions and functional decline of cells. Dermal fibroblasts are located in the dermal layer of the skin and play important roles in maintaining skin structure and function. When the function of dermal fibroblasts is impaired due to oxidative stress, the protective function of the skin may be weakened. This makes the skin more vulnerable to external stimuli and can worsen symptoms of atopic dermatitis. The resulting oxidative stress can promote inflammatory reactions. Such inflammatory reactions are one of the major symptoms of atopic dermatitis and become a cause of dermal fibroblasts not functioning properly. Additionally, cell damage due to oxidative stress can worsen damage to the skin barrier. When the skin barrier is damaged, substances that cause allergic reactions can penetrate the skin more easily, which can further worsen symptoms of atopic dermatitis.

In the present invention, the expression vector may include nucleotide sequences encoding CPNE7 protein or peptides consisting of amino acids of SEQ ID NO: 1. The vectors used are not particularly limited as long as they can produce the peptides, but preferably may be plasmid DNA, phage DNA, etc., more preferably commercially developed plasmids (pUC18, pBAD, pIDTSAMRT-AMP, etc.), E. coli-derived plasmids (pYG601BR322, pBR325, pUC118, pUC119, etc.), Bacillus subtilis-derived plasmids (pUB110, pTP5, etc.), yeast-derived plasmids (YEp13, YEp24, YCp50, etc.), phage DNA (Charon4A, Charon21A, EMBL3, EMBL4, λgt10, λgt11, λZAP, etc.), animal virus vectors (retrovirus, adenovirus, vaccinia virus, etc.), insect virus vectors (baculovirus, etc.). Since the expression vector shows different protein expression levels and modifications depending on the host cell, it is preferable to select and use the most suitable host cell for the purpose.

The transformants provided by the present invention can be prepared by introducing the expression vectors provided by the present invention into hosts and transforming them, and can be used to produce the peptides by expressing the polynucleotides contained in the expression vectors. The transformation can be performed by various methods and is not particularly limited as long as it can produce the peptides, but CaCl2 precipitation method, Hanahan method that increases efficiency by using dimethyl sulfoxide (DMSO) as a reducing agent in the CaCl2 precipitation method, electroporation, calcium phosphate precipitation method, protoplast fusion method, agitation method using silicon carbide fibers, Agrobacterium-mediated transformation method, PEG-mediated transformation method, dextran sulfate, lipofectamine, and dry/inhibition-mediated transformation methods, etc. can be used. Additionally, the hosts used for preparing the transformants are also not particularly limited as long as they can produce the peptides, but may be bacterial cells such as E. coli, Streptomyces, Salmonella typhimurium; yeast cells such as Saccharomyces cerevisiae, Schizosaccharomyces pombe; fungal cells such as Pichia pastoris; insect cells such as Drosophila, Spodoptera Sf9 cells; animal cells such as CHO, COS, NSO, 293, bovine melanoma cells; or plant cells.

The transformants can also be used in methods for producing peptides consisting of amino acids of SEQ ID NO: 1 of the present invention. Specifically, methods for producing peptides consisting of amino acids of SEQ ID NO: 1 of the present invention may include (a) culturing the transformants to obtain cultures; and (b) recovering the peptides of the present invention from the cultures.

The term "culture" in the present invention means a method of growing microorganisms under artificially controlled environmental conditions. In the present invention, methods for culturing the transformants can be performed using methods widely known in the art. Specifically, the culture is not particularly limited as long as it can express and produce peptides consisting of amino acids of SEQ ID NO: 1 of the present invention, but can be cultured continuously in batch processes or fed batch or repeated fed batch processes.

The medium used for culture must meet the requirements of specific strains in appropriate ways while controlling temperature, pH, etc. under aerobic conditions in conventional medium containing appropriate carbon sources, nitrogen sources, amino acids, vitamins, etc. Carbon sources that can be used include mixed sugars of glucose and xylose as main carbon sources, and additionally sugars and carbohydrates such as sucrose, lactose, fructose, maltose, starch, cellulose, oils and fats such as soybean oil, sunflower oil, castor oil, coconut oil, fatty acids such as palmitic acid, stearic acid, linoleic acid, alcohols such as glycerol, ethanol, and organic acids such as acetic acid. These substances can be used individually or as mixtures. Nitrogen sources that can be used include inorganic nitrogen sources such as ammonia, ammonium sulfate, ammonium chloride, ammonium acetate, ammonium phosphate, ammonium carbonate, and ammonium nitrate; amino acids such as glutamic acid, methionine, glutamine and organic nitrogen sources such as peptone, NZ-amine, meat extract, yeast extract, malt extract, corn steep liquor, casein hydrolysate, fish or their decomposition products, defatted soybean cake or their decomposition products. These nitrogen sources can be used alone or in combination. The medium may include potassium dihydrogen phosphate, dipotassium hydrogen phosphate, and corresponding sodium-containing salts as phosphorus sources. Phosphorus sources that can be used include potassium dihydrogen phosphate or dipotassium hydrogen phosphate or corresponding sodium-containing salts. Additionally, inorganic compounds such as sodium chloride, calcium chloride, iron chloride, magnesium sulfate, iron sulfate, manganese sulfate, and calcium carbonate can be used. Finally, essential growth substances such as amino acids and vitamins can be used in addition to the above substances.

Additionally, appropriate precursors can be used in the culture medium. The above-mentioned raw materials can be added to the culture in appropriate ways by batch, fed-batch, or continuous methods during the culture process, but are not particularly limited thereto. The pH of the culture can be controlled by appropriately using basic compounds such as sodium hydroxide, potassium hydroxide, ammonia, or acid compounds such as phosphoric acid or sulfuric acid.

Additionally, antifoaming agents such as fatty acid polyglycol esters can be used to suppress foam formation. To maintain aerobic conditions, oxygen or oxygen-containing gas (e.g., air) is injected into the culture. The temperature of the culture is usually 27°C to 37°C, preferably 30°C to 35°C. Culture continues until the maximum production of the peptides is obtained. For this purpose, it is usually achieved in 10 to 100 hours.

Furthermore, the step of recovering the peptides from the culture can be performed by methods known in the art. Specifically, the recovery method is not particularly limited as long as it can be used for recovery of the produced peptides, but preferably methods such as centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobic and size exclusion), etc. can be used.

According to the present invention, pharmaceutical compositions for wound treatment containing peptides derived from CPNE7 protein can be used in the form of pharmaceutical compositions for treating skin diseases caused by oxidative stress in dermal fibroblasts. Such compositions can be prepared by additionally including appropriate carriers (natural or non-natural carriers), excipients, or diluents commonly used. Specifically, the pharmaceutical compositions can be formulated and used in the form of sterile injectable solutions that can be administered to areas where skin diseases have been induced according to conventional methods. In the present invention, carriers, excipients, and diluents that may be included in the pharmaceutical compositions include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, collagen, etc. When formulating, they can be prepared using diluents or excipients such as commonly used fillers, extenders, binders, wetting agents, disintegrants, surfactants, etc. In particular, sterile aqueous solutions, non-aqueous solvents, suspensions, emulsions, freeze-dried preparations, suppositories, ointments (e.g., root canal sealers, etc.) may be included. As non-aqueous solvents and suspensions, propylene glycol, polyethylene glycol, vegetable oils such as olive oil, injectable esters such as ethyl oleate, etc. can be used. As suppository bases, witepsol, macrogol, tween 61, cacao butter, laurin butter, glycerogelatin, etc. can be used.

The content of the peptides included in the pharmaceutical compositions of the present invention is not particularly limited, but may be included in amounts of 0.0001 to 50% by weight, more preferably 0.01 to 20% by weight based on the total weight of the final composition.

The pharmaceutical compositions of the present invention can be administered in pharmaceutically effective amounts. The term "pharmaceutically effective amount" in the present invention means an amount sufficient to treat or prevent diseases with a reasonable benefit/risk ratio applicable to medical treatment or prevention. The effective dose level can be determined according to factors including the severity of the disease, activity of the drug, age, weight, health, sex of the patient, patient's sensitivity to the drug, administration time of the composition used in the present invention, administration route and excretion rate, treatment period, drugs used in combination or simultaneously with the composition of the present invention, and other factors well known in the medical field. The pharmaceutical compositions of the present invention can be administered alone or in combination with known pharmaceutical compositions for treating skin diseases caused by oxidative stress in dermal fibroblasts. Considering all the above factors, it is important to administer amounts that can obtain maximum effects with minimum amounts without side effects.

The dosage of the pharmaceutical compositions of the present invention can be determined by those skilled in the art considering the purpose of use, severity of disease, patient's age, weight, sex, medical history, or the type of substance used as active ingredient. For example, the pharmaceutical compositions of the present invention can be administered at about 0.1 ng to about 100 mg/kg, preferably 1 ng to about 10 mg/kg per adult. The administration frequency of the compositions of the present invention is not particularly limited, but can be administered once daily or divided into multiple doses. The above dosage does not limit the scope of the present invention in any way.

As another aspect, the present invention provides methods for treating skin diseases caused by oxidative stress in dermal fibroblasts comprising administering the pharmaceutical compositions in pharmaceutically effective amounts to subjects other than humans in whom skin diseases caused by oxidative stress in dermal fibroblasts have developed.

The term "subject" in the present invention can include mammals including mice, livestock, etc. that require treatment for skin diseases caused by oxidative stress in dermal fibroblasts without limitation, but excludes humans from subjects in whom the diseases have developed.

The administration route of the pharmaceutical compositions for treating skin diseases caused by oxidative stress in dermal fibroblasts of the present invention can be administered through any general route as long as it can reach the target tissue. The pharmaceutical compositions of the present invention are not particularly limited, but can be administered through routes such as oral administration, dermal administration, intramuscular injection, intravenous injection, subcutaneous injection, respiratory administration, rectal administration, topical administration, etc. according to the desired purpose.

'Oral administration (intraoral administration)' is a method of ingesting drugs through the mouth, and generally pills, capsules, and liquid forms of drugs can be used. 'Dermal administration' is a method of applying or attaching drugs to the skin, and can be used in forms such as creams, gels, patches, etc. 'Intramuscular injection' is a method of injecting drugs directly into muscle tissue, mainly using syringes and needles. 'Intravenous injection' is a method of injecting drugs directly into blood vessels, using syringes and needles, or venous cannulas. 'Subcutaneous injection' is a method of injecting drugs into fat tissue under the skin, and can be used for drug administration such as insulin. 'Respiratory administration' is a method of delivering drugs to the respiratory system through the mouth or nose, and can be used in forms such as inhalers, gases, vapors, etc. 'Rectal administration' is a method of injecting drugs into the rectum, and can be used in forms such as rectal medications, rectal suppositories, etc. 'Topical administration' is a method of applying drugs directly to areas requiring treatment, and can be used in forms such as creams, gels, patches, etc. according to disease characteristics.

As another aspect, the present invention provides quasi-drug compositions for preventing or improving skin diseases caused by oxidative stress in dermal fibroblasts containing the above peptides.

The term "improvement" in the present invention means all actions that at least reduce parameters related to the condition being treated, for example, the degree of symptoms.

In the present invention, the improvement can be interpreted as meaning all actions that administer pharmaceutical compositions containing the peptides of the present invention as active ingredients to subjects requiring treatment for skin diseases caused by oxidative stress in dermal fibroblasts to promote removal of reactive oxygen species in dermal fibroblasts, thereby improving or benefiting symptoms of skin diseases caused by oxidative stress in dermal fibroblasts.

The term "quasi-drug" in the present invention means products with milder effects than pharmaceuticals among products used for the purpose of diagnosing, treating, improving, alleviating, treating, or preventing diseases in humans or animals. For example, according to the Pharmaceutical Affairs Act, quasi-drugs are those excluding products used for pharmaceutical purposes, including fiber and rubber products used for human and animal disease treatment or prevention, products that have mild effects on the human body or do not act directly and are not instruments or machines and similar products, disinfectants and insecticides for preventing infectious diseases, etc.

In the present invention, the types or formulations of quasi-drug compositions containing the peptides are not particularly limited, but as examples, may be ointments, patches, powders, gels, sprays, tablets, or sprays, etc.

As another aspect, the present invention provides functional food compositions for preventing or improving skin diseases caused by oxidative stress in dermal fibroblasts containing the above peptides.

The term "food" in the present invention includes meat, sausages, bread, chocolate, candies, snacks, confectionery, pizza, ramen, other noodles, gums, dairy products including ice cream, various soups, beverages, teas, drinks, alcoholic beverages, vitamin complexes, functional foods, and health foods, and includes all foods in the conventional sense.

The functional food refers to food with high medical and therapeutic effects processed to efficiently exhibit biological regulatory functions in addition to nutritional supply, which is the same term as food for special health use (FoSHU). Here, "functional" means obtaining useful effects for health purposes such as regulating nutrients for human body structure and function or physiological actions. Foods of the present invention can be manufactured by methods commonly used in the art, and during manufacturing, they can be manufactured by adding raw materials and components commonly added in the art. Additionally, the formulation of the foods can also be manufactured without limitation as long as it is a formulation recognized as food. Food compositions of the present invention can be manufactured in various formulations and, unlike general drugs, have the advantage of being made from food materials and having no side effects that may occur with long-term use of drugs. They have excellent portability, so foods of the present invention can be consumed as supplements to enhance effects of preventing or improving skin diseases caused by oxidative stress in dermal fibroblasts.

Health food means food with more active health maintenance or promotion effects compared to general food, and health supplement food means food for health supplementation purposes. Occasionally, the terms functional food, health food, and health supplement food can be used interchangeably.

Specifically, the functional foods can be foods manufactured by adding the peptides of the present invention to food materials such as beverages, teas, spices, gums, confectionery, or manufactured as capsules, powders, suspensions, etc. When consumed, they mean bringing about specific health effects, but unlike general drugs, they have the advantage of being made from food materials and having no side effects that may occur with long-term use of drugs.

Since the food compositions of the present invention can be consumed daily, they can be very usefully used as they can be expected to have high effects for preventing or improving skin diseases caused by oxidative stress in dermal fibroblasts.

The food compositions may additionally include physiologically acceptable carriers, and the types of carriers are not particularly limited, and any carrier commonly used in the art can be used.

Additionally, the food compositions may include additional components commonly used in food compositions that can improve smell, taste, vision, etc. For example, they may include vitamins A, C, D, E, B1, B2, B6, B12, niacin, biotin, folate, pantothenic acid, etc. They may also include minerals such as zinc (Zn), iron (Fe), calcium (Ca), chromium (Cr), magnesium (Mg), manganese (Mn), copper (Cu), chromium (Cr), etc. They may also include amino acids such as lysine, tryptophan, cysteine, valine, etc.

Additionally, the food compositions may include food additives such as preservatives (potassium sorbate, sodium benzoate, salicylic acid, sodium dehydroacetate, etc.), disinfectants (bleaching powder and high-grade bleaching powder, sodium hypochlorite, etc.), antioxidants (butylhydroxyanisole (BHA), butylhydroxytoluene (BHT), etc.), coloring agents (tar dyes, etc.), color developers (sodium nitrite, sodium acetate, etc.), bleaching agents (sodium sulfite), seasonings (MSG monosodium glutamate, etc.), sweeteners (dulcin, cyclamate, saccharin, sodium, etc.), fragrances (vanillin, lactones, etc.), leavening agents (alum, potassium hydrogen D-tartrate, etc.), fortifiers, emulsifiers, thickeners (starch syrup), coating agents, gum bases, antifoaming agents, solvents, improvers, etc. The additives can be selected according to food types and used in appropriate amounts.

The peptides of the present invention can be added as is or used together with other foods or food components, and can be appropriately used according to conventional methods. The mixing amount of active ingredients can be appropriately determined according to their purpose of use (prevention, health, or therapeutic treatment). Generally, when manufacturing foods or beverages, the food compositions of the present invention can be added in amounts of 50 parts by weight or less, specifically 20 parts by weight or less relative to the food or beverage. However, when consumed for long periods for health and hygiene purposes, they may contain amounts below the above range, and since there are no safety issues, active ingredients can also be used in amounts above the above range.

As one example of the food compositions of the present invention, they can be used as health beverage compositions, and in this case, they may contain various flavoring agents or natural carbohydrates as additional components like conventional beverages. The aforementioned natural carbohydrates may be monosaccharides such as glucose and fructose; disaccharides such as maltose and sucrose; polysaccharides such as dextrin and cyclodextrin; sugar alcohols such as xylitol, sorbitol, erythritol, etc. Sweeteners may use natural sweeteners such as thaumatin and stevia extracts; synthetic sweeteners such as saccharin and aspartame, etc. The ratio of natural carbohydrates can generally be about 0.01 to 0.04 g, specifically about 0.02 to 0.03 g per 100 mL of the health beverage composition of the present invention.

In addition to the above, health beverage compositions may contain various nutrients, vitamins, electrolytes, flavoring agents, coloring agents, pectic acid, salts of pectic acid, alginic acid, salts of alginic acid, organic acids, protective colloidal thickeners, pH adjusters, stabilizers, preservatives, glycerin, alcohol, or carbonating agents, etc. Additionally, they may contain pulp for manufacturing natural fruit juices, fruit juice beverages, or vegetable beverages. These components can be used independently or in combination. The ratio of such additives is not critically important, but is generally selected in the range of 0.01 to 0.1 parts by weight per 100 parts by weight of the health beverage composition of the present invention.

The food compositions of the present invention can contain various weight percentages as long as they can exhibit skin aging inhibition or wound treatment effects including peptides derived from CPNE7 protein, but specifically may contain the peptides of the present invention in 0.00001 to 100% by weight or 0.01 to 80% by weight relative to the total weight of the food composition, but are not limited thereto.

### [Advantageous Effects]

The compositions for inhibiting skin aging or treating wounds containing peptides derived from CPNE7 protein according to embodiments of the present invention can promote synthesis of Type I Collagen and Type III Collagen involved in promoting skin elasticity and preventing aging in fibroblasts within the dermal layer of the skin.

The compositions for inhibiting skin aging or treating wounds containing peptides derived from CPNE7 protein according to other embodiments of the present invention can promote synthesis of Type IV Collagen involved in promoting skin elasticity and preventing aging in the basement membrane of the dermo-epidermal junction of the skin.

The compositions for inhibiting skin aging or treating wounds containing peptides derived from CPNE7 protein according to yet other embodiments of the present invention aim to provide peptides and their uses that promote regeneration of the epidermal layer and prevent aging in keratinocytes within the epidermal layer of the skin.

The compositions for inhibiting skin aging or treating wounds containing peptides derived from CPNE7 protein according to yet other embodiments of the present invention can promote synthesis of Filaggrin and Involucrin involved in promoting skin moisturization and preventing aging in keratinocytes within the epidermal layer of the skin.

The compositions for inhibiting skin aging or treating wounds containing peptides derived from CPNE7 protein according to yet other embodiments of the present invention can reduce expression of MMP-1 involved in preventing skin aging by inhibiting collagen degradation in the epidermal and dermal layers of the skin.

The effects of the present invention are not limited to those mentioned above, and other effects not mentioned will be clearly understood by those with ordinary skill in the technical field to which the present invention belongs from the description below.

### [Description of Drawings]

Figure 1 shows the results of measuring the peptides of the present invention at various concentrations after 48 hours of treatment, followed by dispensing WST-8 solution into the culture medium at a 10:1 ratio, light-shielding, reacting for 2 hours under 37°C incubator conditions, and then measuring at 450nm absorbance with a microplate.
Figure 2 (A) shows the confirmation of Type I Collagen protein formation after treating the peptide (SEQ ID NO: 96) of the present invention at various concentrations for 7 days, and Figure 2 (B) shows the results of confirming the EC50 value according to Type I Collagen protein formation after treating the peptide (SEQ ID NO: 96) of the present invention at 100 uM 7 times over 7 days.
Figure 3a is a graph comparing the Type I Collagen protein expression levels for the peptides in Table 13.
Figure 3b shows a comparison of collagen deposition levels with the control group after treating the peptide (SEQ ID NO: 96) of the present invention at a concentration of 100 uM at 24-hour intervals for 7 days, where (A) shows the Confocal Microscope (Type I Collagen) analysis results and (B) shows the analysis results using the Confocal Microscope Z-Stack Imaging program.
Figure 3c shows the confirmation of alpha-smooth muscle actin (α-SMA) synthesis efficacy of the peptides of the present invention in hDFs (N=3).
Figure 4 shows the results of confirming human-derived dermal fibroblast collagen formation ability upon multiple treatments of the peptides of the present invention.
Figure 5 shows the results of confirming the penetration of the peptides of the present invention into human-derived three-dimensional skin tissue.
Figure 6a shows the results of confirming the efficacy of the peptides of the present invention in human-derived three-dimensional skin tissue.
Figure 6b shows the confirmation of peptide collagen formation ability in Neoderm-ED skin tissue (Ex vivo).
Figure 7 shows the results of confirming the keratinocyte proliferation efficacy of the peptides of the present invention.
Figure 8 shows the results of confirming the efficacy according to skin treatment with the peptides of the present invention.
Figure 9 shows the results of confirming (A) reactive oxygen species reduction effects and (B) anti-photodamage effects according to treatment with the peptides of the present invention.
Figure 10 shows the results of confirming the melanin secretion reduction efficacy according to concentration-dependent treatment with the peptides of the present invention.
Figure 11 shows the results of confirming the migration efficacy of the peptides of the present invention in keratinocytes and hDFs.

### [Modes of the Invention]

The purposes and effects of the present invention, and the technical configurations for achieving them, will become clear by referring to the embodiments described in detail below along with the accompanying drawings. In describing the present invention, when it is determined that detailed descriptions of known functions or configurations may unnecessarily obscure the gist of the present invention, such detailed descriptions will be omitted. The terms described below are terms defined considering the functions in the present invention, and may vary according to the intentions or practices of users and operators.

However, the present invention is not limited to the embodiments disclosed below but may be implemented in various different forms. The present embodiments are provided only to make the disclosure of the present invention complete and to completely inform those with ordinary skill in the technical field to which the present invention belongs of the scope of the invention. The present invention is defined only by the scope of the claims. Therefore, the definition should be made based on the content throughout this specification.

The embodiments of the present invention will be described in detail below.

Example 1. Synthesis of Peptides Derived from CPNE7 Protein

The present inventors synthesized peptides derived from CPNE7 protein (SEQ ID NO: 1) by the 9-fluorenylmethyloxycarbonyl (Fmoc) method and synthesized peptides of each group by substituting amino acids of the synthesized peptides (Tables 1 to 12).

N-KYQRRKKNKY-C (SEQ ID NO: 1)

First, Group 1 peptides were synthesized by substituting amino acids 5 to 7 of the peptide of SEQ ID NO: 1 or the peptide of SEQ ID NO: 1 with lysine or arginine (Table 1).

**[Table 1]**

| Group 1 Peptides | |
|---|---|
| SEQ ID NO | Amino Acid Sequence (N-C) |
| 1 | KYQRRKKNKY |
| 2 | KYQRRKRNKY |
| 3 | KYQRRRKNKY |
| 4 | KYQRRRRNKY |
| 5 | KYQRKKKNKY |
| 6 | KYQRKRKNKY |
| 7 | KYQRKKRNKY |
| 8 | KYQRKRRNKY |

Next, Group 2 peptides were synthesized by substituting amino acids 5 to 7 of the peptide of SEQ ID NO: 1 with lysine or arginine and substituting amino acid 8 with serine (Table 2).

**[Table 2]**

| Group 2 Peptides | |
|---|---|
| SEQ ID NO | Amino Acid Sequence (N-C) |
| 9 | KYQRRKKSKY |
| 10 | KYQRRKRSKY |
| 11 | KYQRRRKSKY |
| 12 | KYQRRRRSKY |
| 13 | KYQRKKKSKY |
| 14 | KYQRKRKSKY |
| 15 | KYQRKKRSKY |
| 16 | KYQRKRRSKY |

Next, Group 3 peptides were synthesized by substituting amino acids 5 to 7 of the peptide of SEQ ID NO: 1 with lysine or arginine and substituting amino acid 9 with tyrosine (Table 3).

**[Table 3]**

| Group 3 Peptides | |
|---|---|
| SEQ ID NO | Amino Acid Sequence (N-C) |
| 17 | KYQRRKKNYK |
| 18 | KYQRRKRNYK |
| 19 | KYQRRRKNYK |
| 20 | KYQRRRRNYK |
| 21 | KYQRKKKNYK |
| 22 | KYQRKRKNYK |
| 23 | KYQRKKRNYK |
| 24 | KYQRKRRNYK |

Next, Group 4 peptides were synthesized by substituting amino acids 5 to 7 of the peptide of SEQ ID NO: 1 with lysine or arginine, substituting amino acid 8 with serine, substituting amino acid 9 with tyrosine, and substituting amino acid 10 with lysine (Table 4).

**[Table 4]**

| Group 4 Peptides | |
|---|---|
| SEQ ID NO | Amino Acid Sequence (N-C) |
| 25 | KYQRRKKSYK |
| 26 | KYQRRKRSYK |
| 27 | KYQRRRKSYK |
| 28 | KYQRRRRSYK |
| 29 | KYQRKKKSYK |
| 30 | KYQRKRKSYK |
| 31 | KYQRKKRSYK |
| 32 | KYQRKRRSYK |

Next, Group 5 peptides were synthesized by substituting amino acid 3 of the peptide of SEQ ID NO: 1 with arginine, substituting amino acid 4 with glutamine, and substituting amino acids 5 to 7 with lysine or arginine (Table 5).

**[Table 5]**

| Group 5 Peptides | |
|---|---|
| SEQ ID NO | Amino Acid Sequence (N-C) |
| 33 | KYRQRKKNKY |
| 34 | KYRQRKRNKY |
| 35 | KYRQRRKNKY |
| 36 | KYRQRRRNKY |
| 37 | KYRQKKKNKY |
| 38 | KYRQKRKNKY |
| 39 | KYRQKKRNKY |
| 40 | KYRQKRRNKY |

Next, Group 6 peptides were synthesized by substituting amino acid 3 of the peptide of SEQ ID NO: 1 with arginine, substituting amino acid 4 with glutamine, substituting amino acids 5 to 7 with lysine or arginine, and substituting amino acid 8 with serine (Table 6).

**[Table 6]**

| Group 6 Peptides | |
|---|---|
| SEQ ID NO | Amino Acid Sequence (N-C) |
| 41 | KYRQRKKSKY |
| 42 | KYRQRKRSKY |
| 43 | KYRQRRKSKY |
| 44 | KYRQRRRSKY |
| 45 | KYRQKKKSKY |
| 46 | KYRQKRKSKY |
| 47 | KYRQKKRSKY |
| 48 | KYRQKRRSKY |

Next, Group 7 peptides were synthesized by substituting amino acid 3 of the peptide of SEQ ID NO: 1 with arginine, substituting amino acid 4 with glutamine, substituting amino acids 5 to 7 with lysine or arginine, substituting amino acid 9 with tyrosine, and substituting amino acid 10 with lysine (Table 7).

**[Table 7]**

| Group 7 Peptides | |
|---|---|
| SEQ ID NO | Amino Acid Sequence (N-C) |
| 49 | KYRQRKKNYK |
| 50 | KYRQRKRNYK |
| 51 | KYRQRRKNYK |
| 52 | KYRQRRRNYK |
| 53 | KYRQKKKNYK |
| 54 | KYRQKRKNYK |
| 55 | KYRQKKRNYK |
| 56 | KYRQKRRNYK |

Next, Group 8 peptides were synthesized by substituting amino acid 3 of the peptide of SEQ ID NO: 1 with arginine, substituting amino acid 4 with glutamine, substituting amino acids 5 to 7 with lysine or arginine, substituting amino acid 8 with serine, substituting amino acid 9 with tyrosine, and substituting amino acid 10 with lysine (Table 8).

**[Table 8]**

| Group 8 Peptides | |
|---|---|
| SEQ ID NO | Amino Acid Sequence (N-C) |
| 57 | KYRQRKKSYK |
| 58 | KYRQRKRSYK |
| 59 | KYRQRRKSYK |
| 60 | KYRQRRRSYK |
| 61 | KYRQKKKSYK |
| 62 | KYRQKRKSYK |
| 63 | KYRQKKRSYK |
| 64 | KYRQKRRSYK |

Next, Group 9 peptides were synthesized by substituting amino acid 3 of the peptide of SEQ ID NO: 1 with lysine, substituting amino acid 4 with glutamine, and substituting amino acids 5 to 7 with lysine or arginine (Table 9).

**[Table 9]**

| Group 9 Peptides | |
|---|---|
| SEQ ID NO | Amino Acid Sequence (N-C) |
| 65 | KYKQRKKNKY |
| 66 | KYKQRKRNKY |
| 67 | KYKQRRKNKY |
| 68 | KYKQRRRNKY |
| 69 | KYKQKKKNKY |
| 70 | KYKQKRKNKY |
| 71 | KYKQKKRNKY |
| 72 | KYKQKRRNKY |

Next, Group 10 peptides were synthesized by substituting amino acid 3 of the peptide of SEQ ID NO: 1 with lysine, substituting amino acid 4 with glutamine, substituting amino acids 5 to 7 with lysine or arginine, and substituting amino acid 8 with serine (Table 10).

**[Table 10]**

| Group 10 Peptides | |
|---|---|
| SEQ ID NO | Amino Acid Sequence (N-C) |
| 73 | KYKQRKKSKY |
| 74 | KYKQRKRSKY |
| 75 | KYKQRRKSKY |
| 76 | KYKQRRRSKY |
| 77 | KYKQKKKSKY |
| 78 | KYKQKRKSKY |
| 79 | KYKQKKRSKY |
| 80 | KYKQKRRSKY |

Next, Group 11 peptides were synthesized by substituting amino acid 3 of the peptide of SEQ ID NO: 1 with lysine, substituting amino acid 4 with glutamine, substituting amino acids 5 to 7 with lysine or arginine, substituting amino acid 9 with tyrosine, and substituting amino acid 10 with lysine (Table 11).

**[Table 11]**

| Group 11 Peptides | |
|---|---|
| SEQ ID NO | Amino Acid Sequence (N-C) |
| 81 | KYKQRKKNYK |
| 82 | KYKQRKRNYK |
| 83 | KYKQRRKNYK |
| 84 | KYKQRRRNYK |
| 85 | KYKQKKKNYK |
| 86 | KYKQKRKNYK |
| 87 | KYKQKKRNYK |
| 88 | KYKQKRRNYK |

Finally, Group 12 peptides were synthesized by substituting amino acid 3 of the peptide of SEQ ID NO: 1 with lysine, substituting amino acid 4 with glutamine, substituting amino acids 5 to 7 with lysine or arginine, substituting amino acid 8 with serine, substituting amino acid 9 with tyrosine, and substituting amino acid 10 with lysine (Table 12).

**[Table 12] [00140]**

| Group 12 Peptides | |
|---|---|
| SEQ ID NO | Amino Acid Sequence (N-C) |
| 89 | KYKQRKKSYK |
| 90 | KYKQRKRSYK |
| 91 | KYKQRRKSYK |
| 92 | KYKQRRRSYK |
| 93 | KYKQKKKSYK |
| 94 | KYKQKRKSYK |
| 95 | KYKQKKRSYK |
| 96 | KYKQKRRSYK |

### Example 2. WST-8 Assay (Cellrix, B1007-500)

Human dermal fibroblasts (hDFs) were dispensed at 0.3×10⁵/well in 96-well plates (using Fibroblast Growth Medium (Lonza, CC-4126) containing 2% FBS, 0.1% Insulin, 0.1% FGF, 0.1% Gentamycin sulfate-Amphotericin) and cultured for 24 hours in an incubator at 37°C, 5% CO₂ conditions.

WST-8 analysis was performed to confirm cytotoxicity of the peptide (SEQ ID NO: 96) in dermal fibroblasts. The peptide was treated on dermal fibroblasts at concentrations of 10, 100 µM, 1, 5, 10 mM respectively. After treating for 48 hours at each concentration, WST-8 solution was dispensed into the culture medium at a 10:1 ratio, light-shielded, reacted for 2 hours under 37°C incubator conditions, and then measured at 450nm absorbance with a microplate.

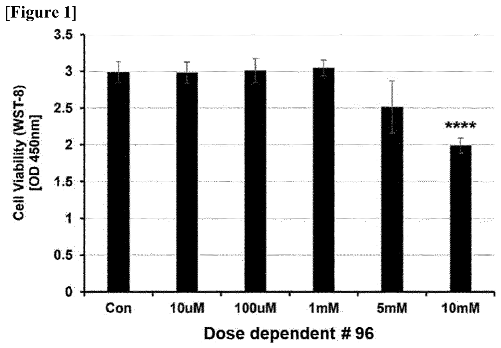

Figure 1 shows the results of measuring the peptide (SEQ ID NO: 96) of the present invention at various concentrations after 48 hours of treatment, followed by dispensing WST-8 solution into the culture medium at a 10:1 ratio, light-shielding, reacting for 2 hours under 37°C incubator conditions, and then measuring at 450nm absorbance with a microplate.

The peptide (SEQ ID NO: 96) of the present invention was treated on dermal fibroblasts at concentrations of 10, 100 µM, 1, 5, 10 mM respectively. After 48 hours, cell viability was quantified through WST-8 analysis. As a result, the peptide (SEQ ID NO: 96) of the present invention showed no cytotoxicity to dermal fibroblasts up to a concentration of 5 mM and showed significant cytotoxicity at a concentration of 10 mM.

### Example 3-1. Confirmation of EC50 Value for Type I Collagen Formation Following Multiple Treatments of Peptides in hDFs

To confirm the Type I collagen synthesis ability following multiple treatments of the peptide (SEQ ID NO: 96) of the present invention, dermal fibroblasts were dispensed at 1×10⁵/6 well plate in 60mm dishes and cultured for 24 hours in an incubator at 37°C, 5% CO₂. The peptide (SEQ ID NO: 96) was treated at concentrations of 1 pM, 10 pM, 100 pM, 1 nM, 10 nM, 1000 nM, 1 µM, 10 µM, 100 µM, 1 mM (total 11 groups) at 24-hour intervals for 7 days.

Subsequently, after washing cells with PBS, proteins were separated and changes in Type I Collagen protein levels were confirmed through Western blot, and comparisons were made through normalization with β-Actin protein levels.

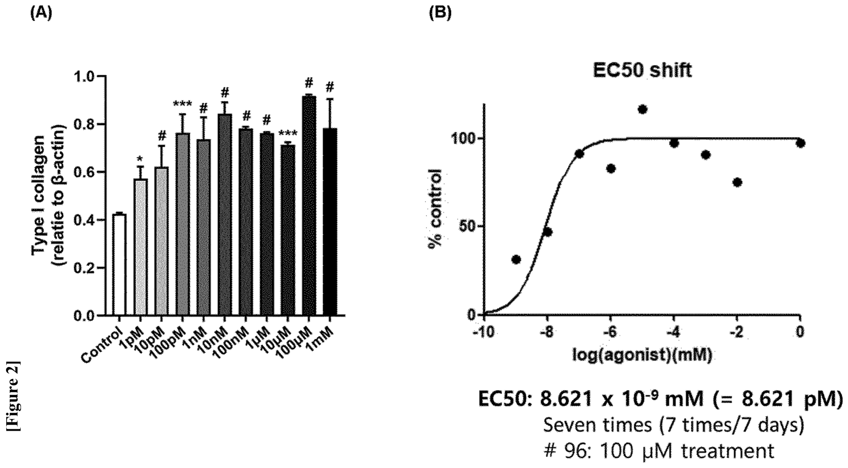

Figure 2 (A) shows the confirmation of Type I Collagen protein formation after treating the peptide (SEQ ID NO: 96) of the present invention at various concentrations for 7 days, and Figure 2 (B) shows the results of confirming the EC50 value according to Type I Collagen protein formation after treating the peptide (SEQ ID NO: 96) of the present invention at 100 µM 7 times over 7 days. Referring to Figure 2, the EC50 value for multiple treatments of the peptide (SEQ ID NO: 96) of the present invention was confirmed to be 8.621 pM, and it can be seen that Type I Collagen expression significantly increased compared to the control group.

### Example 3-2. Confirmation of Type I Collagen Synthesis Ability of Group 1 to 12 Peptides in Human-Derived Dermal Fibroblasts

To confirm the Type I collagen synthesis ability of peptides in each group, dermal fibroblasts were dispensed at 1×10⁵/6 well plate in 60mm dishes and cultured for 24 hours in an incubator at 37°C, 5% CO₂. Each peptide was treated at 100 µM and cultured for 24 hours. After 24 hours, after washing cells with PBS, proteins were separated and changes in Type I Collagen protein levels were confirmed through Western blot, and comparisons were made through normalization with β-Actin protein levels.

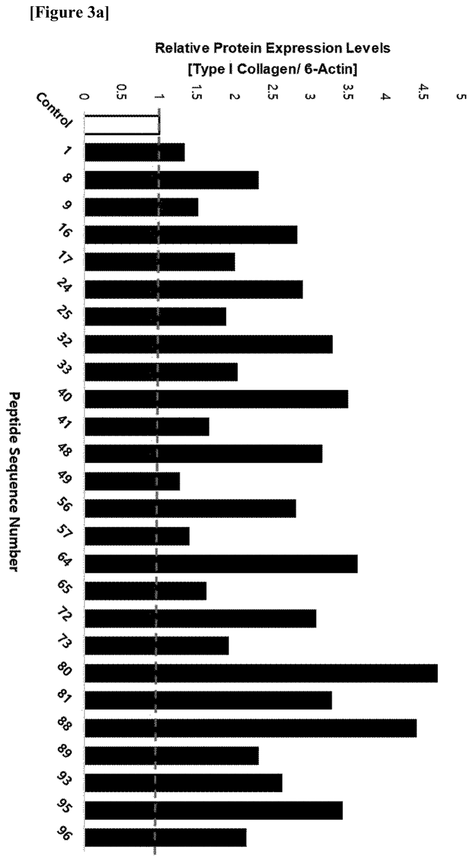

Table 13 shows the results of confirming changes in Type I Collagen protein levels after single treatment of peptides from Groups 1 to 12 of the present invention at a concentration of 100 µM on dermal fibroblasts and 24 hours later. Figure 3a is a graph comparing Type I Collagen protein expression levels for the peptides in Table 13. As can be confirmed through this, it can be seen that the peptides of the present invention show excellent Type I collagen synthesis ability.

| SEQ ID NO | Amino Acid Sequence | Relative Protein Expression levels of Type I Collagen |
|---|---|---|
| Control | - | 1 |
| 1 | KYQRRKKNKY | 1.33516 |
| 8 | KYQRKRRNKY | 2.31151 |
| 9 | KYQRRKKSKY | 1.5182 |
| 16 | KYQRKRRSKY | 2.83006 |
| 17 | KYQRRKKNYK | 1.99857 |
| 24 | KYQRKRRNYK | 2.8985 |
| 25 | KYQRRKKSYK | 1.88521 |
| 32 | KYQRKRRSYK | 3.29178 |
| 33 | KYRQRKKNKY | 2.03416 |
| 40 | KYRQKRRNKY | 3.50524 |
| 41 | KYRQRKKSKY | 1.6575 |
| 48 | KYRQKRRSKY | 3.15834 |
| 49 | KYRQRKKNYK | 1.27334 |
| 56 | KYRQKRRNYK | 2.80845 |
| 57 | KYRQRKKSYK | 1.40067 |
| 64 | KYRQKRRSYK | 3.62404 |
| 65 | KYKQRKKNKY | 1.61994 |
| 72 | KYKQKRRNKY | 3.08218 |
| 73 | KYKQRKKSKY | 1.91764 |
| 80 | KYKQKRRSKY | 4.68996 |
| 81 | KYKQRKKNYK | 3.28704 |
| 88 | KYKQKRRNYK | 4.40885 |
| 89 | KYKQRKKSYK | 2.31899 |
| 93 | KYKQKKKSYK | 2.63127 |
| 95 | KYKQKKRSYK | 3.43182 |
| 96 | KYKQKRRSYK | 2.14839 |

### Example 3-3. Confirmation of Type I Collagen Deposition Following Multiple Administrations of Peptides in hDFs

To confirm the Type I collagen synthesis ability following multiple administrations of the peptide (SEQ ID NO: 96) of the present invention, dermal fibroblasts were dispensed at 1×10⁵/6 well plate in 60mm dishes and cultured for 24 hours in an incubator at 37°C, 5% CO₂. The peptide (SEQ ID NO: 96) and Vitamin C (positive control) were each treated at a concentration of 100 µM at 24-hour intervals for 7 days.

Subsequently, after washing cells with PBS, proteins were separated and changes in Type I Collagen protein levels were confirmed through Confocal Microscope (Type I Collagen).

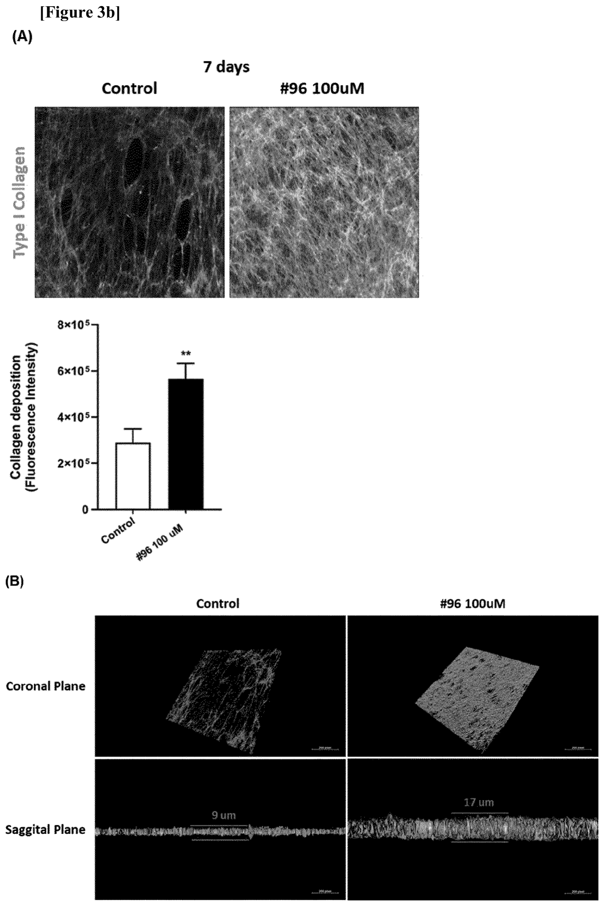

Figure 3b shows a comparison of collagen deposition levels with the control group after treating the peptide (SEQ ID NO: 96) of the present invention at a concentration of 100 µM at 24-hour intervals for 7 days, where (A) shows the Confocal Microscope (Type I Collagen) analysis results and (B) shows the analysis results using the Confocal Microscope Z-Stack Imaging program.

Through this, it was confirmed that on day 7, the amount of Type I Collagen in the ECM (Extracellular Matrix) significantly increased in the peptide (SEQ ID NO: 96) treatment group of the present invention compared to the control group.

### Example 3-4. Confirmation Test of α-SMA Synthesis Efficacy of Peptides in hDFs

To confirm the possibility of promoting wound healing following administration of the peptide (SEQ ID NO: 96) of the present invention, dermal fibroblasts were dispensed at 1.5×10⁵/Confocal Dish and cultured for 24 hours in an incubator at 37°C, 5% CO₂. Peptide (SEQ ID NO: 96) and Vitamin C (positive control) at 100 µM were treated for 48 hours.

Subsequently, after washing cells with PBS, changes in alpha-smooth muscle actin (α-SMA) protein levels were confirmed through Confocal Microscope.

Alpha-smooth muscle actin ("α-SMA") is expressed in fibroblasts of the skin dermis and plays a role in providing structural support to skin tissue and maintaining skin elasticity. When wounds occur, fibroblasts at the wound site express α-SMA to contract the wounded skin. This can reduce wound size and protect wounded skin from tissue damage during the wound healing process. Therefore, when inflammation occurs in the skin, α-SMA increases at inflammatory sites to regulate inflammatory responses, so α-SMA plays an important role in maintaining and stabilizing skin tissue.

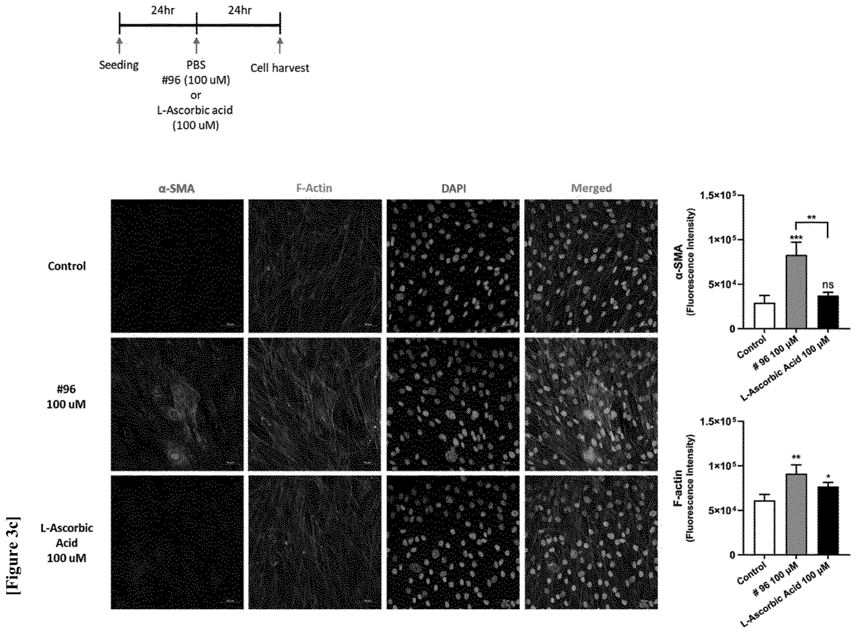

Figure 3c shows the confirmation of alpha-smooth muscle actin (α-SMA) synthesis efficacy of the peptide (SEQ ID NO: 96) in hDFs (N=3). Referring to Figure 3c, it can be seen that α-SMA significantly increases even with just a single treatment of peptide (SEQ ID NO: 96) at 100 µM, indicating wound healing promotion efficacy.

### Example 4. Observation of Cellular Changes Through Optical Microscopy

The collagen synthesis efficacy between Vitamin C, known as a representative substance that increases collagen synthesis ability of dermal fibroblasts in vitro, and the peptide (SEQ ID NO: 96) of the present invention was compared by applying to human-derived dermal fibroblasts. Groups were divided into single application (total 1 time) and multiple application (total 7 times) groups, and peptide (SEQ ID NO: 96) at 100 µM and Vitamin C at 100 µM were each treated for a total of 7 days.

Specifically, dermal fibroblasts were dispensed at 0.3×10⁵/well in 96-well plates and cultured for 24 hours in an incubator at 37°C, 5% CO₂.

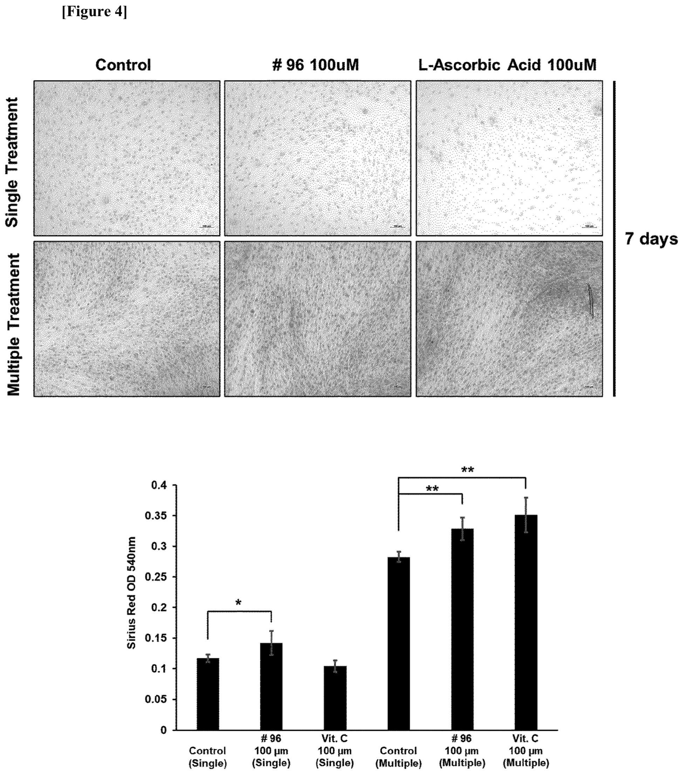

Picro Sirius Red staining is one of the representative collagen staining methods that mainly targets Type I Collagen and Type III Collagen. After treating peptide (SEQ ID NO: 96) of the present invention at 100 µM and Vitamin C at 100 µM for a total of 7 days with single application (total 1 time) and multiple application (total 7 times), Picro Sirius Red staining analysis was performed. When collagen positive for Picro Sirius Red was quantified, it was observed to be higher in the peptide (SEQ ID NO: 96) treatment group of the present invention compared to the control group in the single application group (Figure 4). In the multiple application group, collagen positive for Picro Sirius Red clearly increased much more in the peptide (SEQ ID NO: 96) treatment group of the present invention and the Vitamin C treatment group compared to the control group, and significance was also observed in the quantitative results (Figure 4).

### Example 5-1. Results of Confirming Skin Penetration of Peptides of the Present Invention in Human-Derived Three-Dimensional Skin Tissue

To confirm the possibility of skin penetration of the peptides of the present invention, three-dimensional skin tissue composed of epidermis and dermis was created using human-derived keratinocytes and dermal fibroblasts, and then fluorescence-conjugated peptide (SEQ ID NO: 96) of the present invention at 1 mM was treated. After treatment, the position of fluorescence was continuously observed through confocal microscopy at 1, 6, 12, and 24 hours.

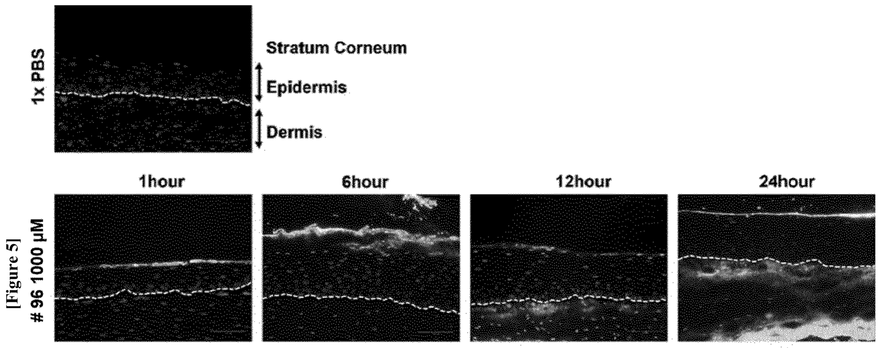

After 1 hour of treatment, the peptide (SEQ ID NO: 96) of the present invention was observed in the stratum corneum, and after 6 hours it was observed in the epidermal layer (Figure 5). It began to be observed in the dermal layer from 12 hours onward, and after 24 hours it was clearly observed that the peptide (SEQ ID NO: 96) of the present invention existed in the dermal layer (Figure 5). Through this, it was confirmed that the peptide (SEQ ID NO: 96) of the present invention can penetrate through the stratum corneum and epidermal layer to the dermal layer.

### Example 5-2. Confirmation of Skin Penetration of Peptides in Human-Derived Three-Dimensional Skin Tissue

FITC fluorescence was attached to the lysine residue of the peptide (SEQ ID NO: 96) of the present invention to create Peptide-FITC using the peptide (SEQ ID NO: 96) of the present invention. Peptide-FITC at 1 mM was treated on human-derived three-dimensional skin tissue (TegoScience, Neoderm-ED) and the skin penetration state of Peptide-FITC was confirmed through confocal microscopy at 1 hour, 6 hours, 12 hours, and 24 hours.

Specifically, to confirm tissue and cytological changes when treating the peptide (SEQ ID NO: 96) of the present invention in human-derived three-dimensional skin tissue, Hematoxylin & Eosin (H&E) staining was performed on tissue sections 24 hours after treating the peptide (SEQ ID NO: 96) of the present invention at 1 mM for analysis.

Normal epidermal layers consist of stratum corneum, stratum lucidum, stratum granulosum, stratum spinosum, and stratum basale from the outside. Millions of new keratinocytes are formed daily in the basal layer, and these do not remain in one place but are continuously pushed outward to the outermost part of the epidermis. While keratinocytes are pushed upward, these cells gradually change into hard keratin. Aged keratinocytes continuously fall off from the surface of human skin, but in aged skin, new keratinocyte formation does not occur well, so it takes more time for the stratum corneum to fall off, causing the stratum corneum to thicken. Therefore, functional decline of keratinocytes causes more dead keratinocytes to accumulate, which can be a direct cause of fine wrinkles and skin roughening.

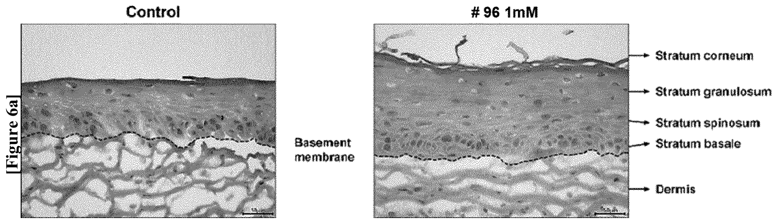

Histological analysis results showed that in the control group, keratinocytes were observed in the basal layer, but the stratum spinosum, stratum granulosum, stratum lucidum, and stratum corneum were not yet properly formed (Figure 6a). In contrast, in the peptide treatment group of the present invention, normal histological characteristics of the epidermis were well observed (Figure 6a). This means that the peptides of the present invention can promote keratinization through enhancing keratinocyte function.

### Example 5-3. Confirmation of Peptide Collagen Formation Ability in Human-Derived Three-Dimensional Skin Tissue (Ex vivo)

The collagen synthesis efficacy between Vitamin C and the peptides of the present invention was compared by applying to human-derived dermal fibroblasts. To confirm peptide collagen formation ability in skin tissue of the peptides of the present invention, three-dimensional skin tissue composed of epidermis and dermis was created (Neoderm-ED) using human-derived keratinocytes and dermal fibroblasts, and then Vitamin C at 1,000 µM and the peptide (SEQ ID NO: 96) of the present invention at 10 µM, 100 µM, 300 µM, 1,000 µM each were treated twice. After treatment, collagen formation ability for negative control, positive control, and treatment groups was confirmed at 48-hour intervals using the Masson Trichrome Staining method.

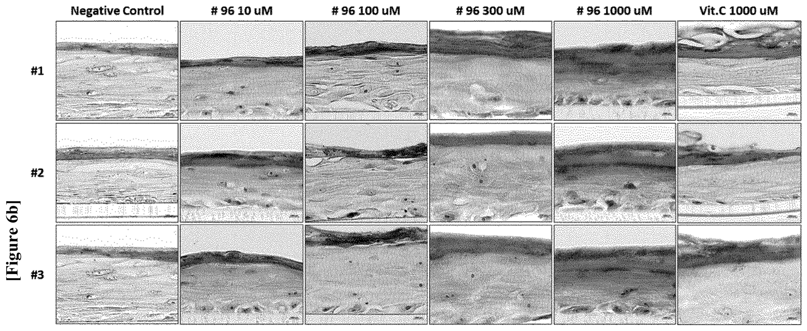

Figure 6b shows the confirmation of peptide collagen formation ability in Neoderm-ED skin tissue (Ex vivo). Confirming Figure 6b, excellent collagen formation ability according to treatment with the peptides of the present invention can be confirmed. Specifically, it can be confirmed that collagen (blue) deposition amount increases dose-dependently in the peptide (SEQ ID NO: 96) treatment group compared to the negative control (NC group).

### Example 5-4. Test for Confirming Peptide Skin Efficacy in Human-Derived Three-Dimensional Skin Tissue

To confirm the proliferation efficacy of keratinocytes in the basal layer of the peptides of the present invention, immunofluorescence staining analysis of Ki-67, a representative cell division and proliferation marker, was performed. For this, the peptide (SEQ ID NO: 96) of the present invention at 1 mM was treated on human-derived three-dimensional skin tissue (TegoScience, Neoderm-ED) and cultured for 24 hours in an incubator at 37°C, 5% CO₂. Then, each human-derived three-dimensional skin tissue was fixed and frozen sectioned. The sectioned tissues were stained with Hematoxylin & Eosin for comparative analysis using optical microscopy (confocal microscopy).

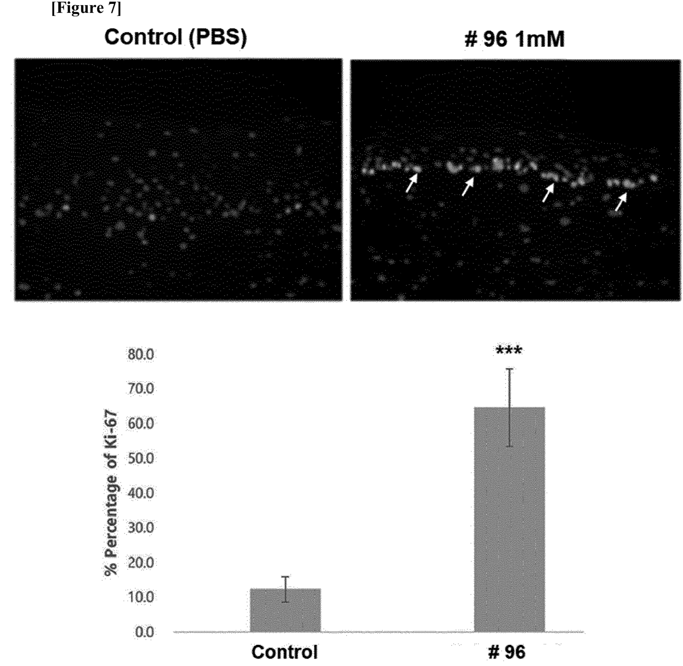

Referring to Figure 7, it can be confirmed that the number of cells positive for Ki-67 in keratinocytes within the basal layer increased compared to the control group 24 hours after treating human-derived three-dimensional skin tissue with the peptide (SEQ ID NO: 96) of the present invention at 1 mM. Quantitative results for the ratio of Ki-67 positive cells showed that the peptide (SEQ ID NO: 96) treatment group of the present invention was 64.6 (±11.1%), which was a 5.3-fold increase compared to the control group's 12.2 (±3.6)% (Figure 7). This can be interpreted as the peptides of the present invention promoting cell division and proliferation of keratinocytes.

### Example 5-5. Test for Confirming Peptide Skin Efficacy in Human-Derived Three-Dimensional Skin Tissue

To confirm the skin efficacy of the peptides of the present invention, expression patterns of Type IV Collagen, MMP-1, Filaggrin, and Involucrin were compared through immunofluorescence analysis 24 hours after treating human-derived three-dimensional skin tissue with the peptides of the present invention at 1 mM.

Type IV Collagen is mainly expressed in the epidermal basement membrane at the dermo-epidermal junction. The basement membrane has anatomical functions of joining the epidermis and the underlying dermis structure, facilitating permeation of liquid substances between the epidermis and dermis while simultaneously performing a defensive role controlling permeation of inflammatory cells or tumor cells. According to recent studies, changes in the basement membrane occur in aged skin, and it has been revealed that Type IV Collagen decreases at this time. Such changes can cause not only deformation of keratinocytes but also increases in MMP, a collagen-degrading enzyme.

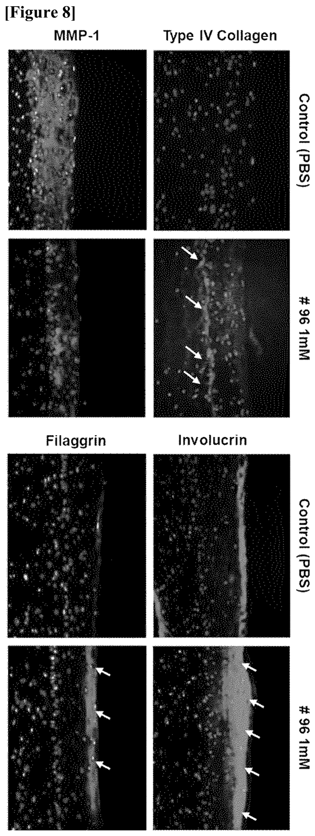

Referring to Figure 8, it can be confirmed that the peptide (SEQ ID NO: 96) of the present invention reduced MMP-1 expression in the epidermis and dermis of human-derived three-dimensional skin tissue and increased Type IV Collagen expression in the basement membrane. Maintaining skin moisture is a basic condition for maintaining healthy skin, and the stratum corneum maintains moisture through natural moisturizing factors (Natural Moisturizing Factor) such as Filaggrin or Involucrin, lipid layers existing between keratinocytes, and sebum secreted from sebaceous glands.

Filaggrin acts as an adhesive connecting the outer membrane of keratinocytes and keratin intermediate microfibers, and Involucrin has important functions in forming and maintaining the function of keratinocyte cell membranes that act as physical barriers for the skin. Decreased expression of these can cause skin diseases such as psoriasis and atopic dermatitis due to impaired skin barrier function. In this regard, referring to Figure 8, it can be confirmed that the peptides of the present invention increase the expression levels of natural moisturizing factors Filaggrin and Involucrin in the epidermis of human-derived three-dimensional skin tissue (Figure 8).

### Example 6-1. Confirmation of Anti-photodamage Efficacy of Peptides in Neoderm-ME Tissue

To confirm the anti-photodamage efficacy of the peptides of the present invention, UV-B radiation was applied to human-derived three-dimensional skin tissue composed of keratinocytes and melanocytes. Subsequently, 48 hours after treating the peptides of the present invention at 100 µM, expression patterns of DCFDA staining, Filaggrin, and Involucrin were compared through immunofluorescence analysis.

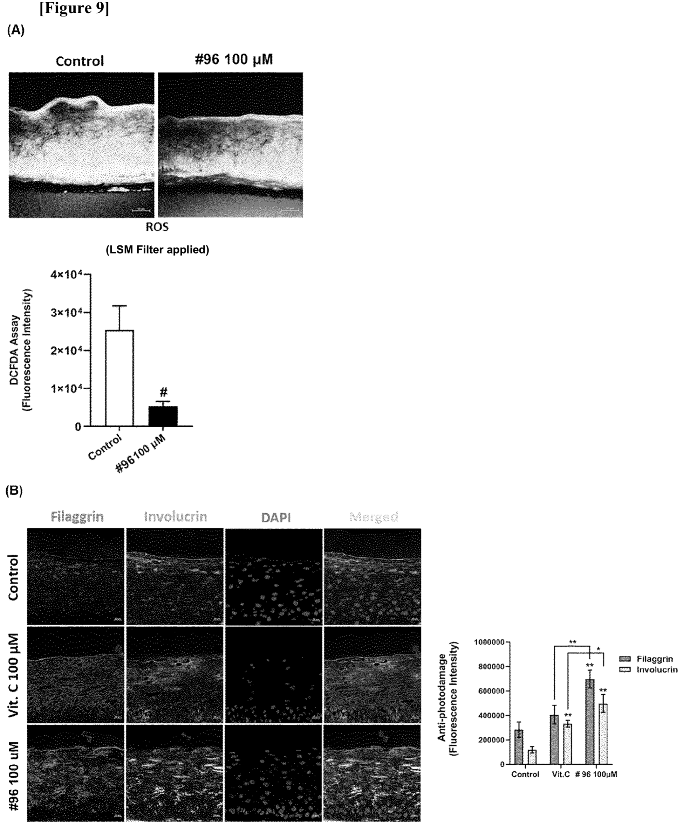

Figure 9 shows the results of confirming (A) reactive oxygen species reduction effects and (B) anti-photodamage effects according to treatment with peptide (SEQ ID NO: 96), confirming that the peptide (SEQ ID NO: 96) treatment group provides significant skin damage reduction effects according to UV-B irradiation.

### Example 6-2. Confirmation of Skin Whitening Efficacy Using Neoderm-ME

To confirm the skin whitening efficacy of the peptides of the present invention, UV-B radiation was applied to human-derived three-dimensional skin tissue composed of keratinocytes and melanocytes. Subsequently, 48 hours after treating the peptides of the present invention at 30 µM, 100 µM, 300 µM, 1000 µM, comparisons were made through Melanin Content Assay and Fontana Masson Staining analysis methods.

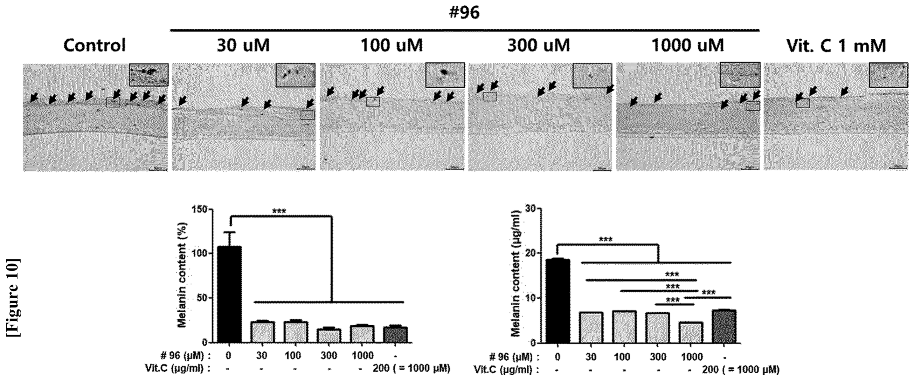

Figure 10 shows the confirmation of melanin secretion reduction efficacy according to concentration-dependent treatment with peptide (SEQ ID NO: 96), confirming that significant melanin secretion reduction occurs at concentrations above 30 µM.

### Example 7. Test for Confirming Migration Efficacy of SEQ ID NO: 96 in Keratinocytes and hDFs

To confirm the possibility of cell migration and wound healing promotion following administration of the peptide (SEQ ID NO: 96) of the present invention, keratinocytes and dermal fibroblasts were dispensed at 1.5×10⁵/Transwell, treated with peptide (SEQ ID NO: 96) at 100 µM, and cultured for 48 hours in an incubator at 37°C, 5% CO₂.

Subsequently, after washing cells with PBS, Crystal violet staining was performed to confirm changes in cell migration through Light Microscope.

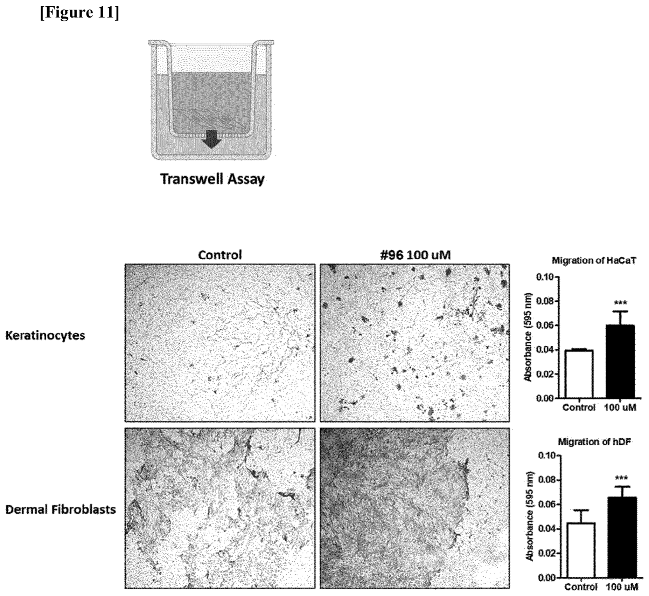

Figure 11 shows the confirmation of cell migration efficacy of the peptides of the present invention in keratinocytes and hDFs, confirming that cell migration is promoted by the peptides of the present invention in human dermal fibroblasts and keratinocytes, indicating wound healing promotion efficacy.

The present specification and drawings have disclosed preferred embodiments of the present invention, and although specific terms have been used, these were used only in a general sense to easily explain the technical content of the present invention and help understanding of the invention, not to limit the scope of the present invention. It is obvious to those with ordinary skill in the technical field to which the present invention belongs that other modifications based on the technical spirit of the present invention can be implemented in addition to the embodiments disclosed herein.

## Claims

1. A composition for treating skin aging or skin wounds comprising a peptide consisting of the amino acid sequence of General Formula 1 below:
K-Y-R1-R2-R3-R4-R5-R6-R7-R8 (General Formula 1)
In General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are lysine (K) or tyrosine (Y).

2. The composition according to Claim 1, **characterized in that** the composition promotes activity of Type I Collagen, Type III Collagen, Type IV Collagen, Ki-67, Filaggrin, or Involucrin, or inhibits activity of MMP-1.

3. The composition according to Claim 1, **characterized in that** the peptide is any one amino acid sequence among SEQ ID NOs: 1 to 96.

4. A polynucleotide encoding the peptide of Claim 1.

5. An expression vector containing the polynucleotide of Claim 4.

6. The composition according to Claim 1, **characterized in that** the composition comprises a polypeptide in which the peptide is repeatedly linked.

7. A composition for preventing, improving, or treating skin aging through promoting skin regeneration, elasticity, and moisturization comprising a peptide consisting of any one amino acid sequence among SEQ ID NOs: 1 to 96.

8. A quasi-drug composition for preventing or improving skin aging through promoting skin regeneration, elasticity, and moisturization comprising a peptide consisting of any one amino acid sequence among SEQ ID NOs: 1 to 96.

9. A cosmetic composition for preventing or improving skin aging through promoting skin regeneration, elasticity, and moisturization comprising a peptide consisting of any one amino acid sequence among SEQ ID NOs: 1 to 96.

10. A functional food composition for preventing or improving skin aging through promoting skin regeneration, elasticity, and moisturization comprising a peptide consisting of any one amino acid sequence among SEQ ID NOs: 1 to 96.

11. The skin treatment composition according to Claim 7, **characterized by** additionally comprising pharmaceutically acceptable carrier, excipient, or diluent.

12. A method for preventing, improving, or treating skin aging through promoting skin regeneration, elasticity, and moisturization by administering the composition of Claim 7 to subjects other than a human.

13. The composition according to Claim 1, wherein the skin wounds are caused by trauma, diabetes, or bedsores.
